**Europäisches Patentamt**

**European Patent Office**

**Office européen des brevets**

(19)

(11) Veröffentlichungsnummer: **0 196 393**
**B1**

(12) # EUROPÄISCHE PATENTSCHRIFT

(45) Veröffentlichungstag der Patentschrift:
**14.06.89**

(21) Anmeldenummer: **85810144.7**

(22) Anmeldetag: **01.04.85**

(51) Int. Cl.⁴: **A 61 N 1/36**

(54) **Elektrostimulationsgerät, insbesondere zur Skoliosebehandlung.**

(43) Veröffentlichungstag der Anmeldung:
**08.10.86 Patentblatt 86/41**

(45) Bekanntmachung des Hinweises auf die Patenterteilung:
**14.06.89 Patentblatt 89/24**

(84) Benannte Vertragsstaaten:
**CH DE FR GB LI**

(56) Entgegenhaltungen:
**FR-A- 2 351 671**
**FR-A- 2 536 286**

(73) Patentinhaber: **OSCOBAL AG, Bohnackerweg 1,
CH-2545 Selzach (CH)**

(72) Erfinder: **Schmitt, Otmar, In der Kohlwies,
D-6680 Münchwies (DE)**
Erfinder: **Mittelmeier, Heinz, Am Gedünner 28,
D-6650 Homburg-Schwarzenbach (DE)**

(74) Vertreter: **Seehof, Michel, c/o AMMANN
PATENTANWAELTE AG BERN Schwarztorstrasse 31,
CH-3001 Bern (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung bezieht sich auf ein Elektrostimulationsgerät, insbesondere zur Skoliosebehandlung, mit mindestens einem Kanal für ein Elektrodenpaar, und einer elektrischen Schaltung zur Einstellung der Dauer, Amplitude und Frequenz der Einzelimpulse sowie der Pausen zwischen den Einzelimpulsen und des zeitlichen Verlaufs von Impulsserien, wobei die Amplitude der einzelnen Rechteckimpulse in einer Anfangsphase zunimmt, dann einen Plateauwert annimmt und anschliessend wieder abnimmt. Ein solches Gerät ist aus der US-A-4326 534 bekannt. Außerdem sind in zwei Nachfolgepatenten, den US-A-4342 317 und 4408 609 des gleichen Erfinders, weitere Ausführungsformen eines solchen Gerätes beschrieben.

Gerade bei der Skoliose, für welche das oben genannte Gerät insbesondere gedacht ist, d. h. bei einer solchen Erkrankung der Wirbelsäule, welche vor allem durch Seitverbiegung und -verdrehung gekennzeichnet ist, liegt eine krankhafte Verschiebung des Muskelfaser-Musters vor, welche einer besonderen Ansprache durch Elektrostimulation bedarf, um dieselbe wieder zur Norm zurückzuführen, Untersuchungen der letzten Zeit haben ergeben, daß das normale Verteilungsmuster der Muskelfasern mit ca. 64% Tonusfasern und 36% Zuckungsfasern, bei der Skoliose zu Gunsten der Tonusfasern verschoben ist. Diese Tonusfasern, welche die Aufgabe haben, einen gewissen Dauerspannungszustand der Muskulatur aufrechtzuerhalten, sprechen auf elektrophysiologische Stimulation weniger an, indem bei ihnen die Membraneigenschaften verändert sind. Damit stehen bei diesen Patienten weniger Zuckungsfasern zur Verfügung, welche auf elektrophysiologische Stimulationsreize gut ansprechen und damit für das Ausmass der muskulären Einwirkung auf die Wirbelsäulenverkrümmung von entscheidender Bedeutung sind. Es ist aber in neueren Tierversuchen bekannt geworden, daß es möglich ist, durch Hochfrequenzstimulation die Zahl der Tonusfasern zurückzudrängen und das gestörte Gleichgewicht zu Gunsten der Zuckungsfasern neu zu regulieren.

Grundsätzlich sollte ferner diese Therapie, wie jede andere auch, einen optimalen Erfolg bei kürzester Zeitdauer der Behandlung gewährleisten. Werden jedoch zu große Stromstärken, welche für den Grad der Muskelstimulation und damit des Behandlungserfolges bedeutungsvoll sind, angewandt, entstehen Hautschmerzen, welche jedoch in Abhängigkeit von verschiedenen Stromparametern in unterschiedlicher Weise auftreten. Andererseits entstehen bei Langzeitanwendungen entzündliche Hautreizungen an den Elektroden. Es kommt daher darauf an, optimale Stromimpulse in einer insgesamt relativ kurzen Behandlungszeit einzuleiten.

Das in den drei oben zitierten Patentschriften beschriebene Gerät wird, nach einer kurzen Angewöhnungszeit, für die sogenannte Nachtstimulation angewandt, die jedoch nach vorliegender Erkenntnis unzureichend ist, da – um den Schlaf nicht zu stören – nur mit relativ niedrigen Stromstärken gearbeitet werden kann, da erhöhte Stromreize zu so starken Muskelzuckungen führen, daß die Patienten davon erwachen. Das lange Aufliegen der Elektroden führt außerdem zu den bereits erwähnten Hautreizungen. Außerdem werden im erwähnten Gerät an- und abschwellende Impulsserien erzeugt, die mit sehr kurzen Impulsen von 10 µs beginnen, die dann mit zunehmender Impulsseriendauer und Amplitude länger werden und während des Impulsplateaus die größte Impulslänge von 200 µs erreichen. Infolge der anfänglich sehr kurzen Einzelimpulslängen ist dies vom membran-physiologischen Gesichtspunkt her eine ungünstige Impulsserienform. Da die Impulse nur im Bereich der seitlichen Interkostalmuskulatur angesetzt werden, reichen sie offenbar nicht aus, im Bereich der äußerst wichtigen paravertebralen Muskulatur, d. h. unmittelbar neben der Wirbelsäule, den entsprechenden Effekt zu erzielen und sind insbesondere nicht in der Lage, hier auch die wesentliche indirekte Reizung über die Interkostalnerven zu bewerkstelligen. Dies führt dazu, daß mit Hilfe von 2-Kanalstimulatoren gearbeitet wurde, siehe die ebenfalls angegebenen Folgepatente, die entweder mit überlappenden Impulsserien oder aber alternierenden Impulsserien arbeiten. Hier sind dann zum Erreichen der verschiedenen Muskelgruppen einer Krümmung jeweils zwei Kanäle erforderlich. Dies hat aber dann den Nachteil, daß der zweite Kanal nicht mehr für die Gegenschwingung der meistens S-förmigen Skoliosen zur Verfügung steht. Das heißt aber auch, daß die Muskelstimulation nur auf einen Teil der Skoliose eingeschränkt wird. Außerdem bewirken die kurzen Impulse, daß nur die oberflächlich liegenden Muskelfasern der Muskelgruppen erreicht werden, welche die niedrigste Reizschwelle besitzen, nicht dagegen die Kernzonen mit hoher Reizschwelle.

Außerdem benutzen diese und auch andere sich auf dem Markt befindliche Geräte, beispielsweise gemäß DE-A-3341 732, das Niederfrequenzkonzept, d. h. Impulsfrequenzen von etwa 30 Hz, um keine Muskelermüdungen zu provozieren. Es ist jedoch bekannt, daß ein Muskel, der beim Training nicht ermüdet, nicht ausreichend trainiert wird. Wesentlich dabei ist, daß dann Erholungspausen eingelegt werden. Es wurde bekannt, daß die niedrigfrequenten Impulse die Tonusfasern der Muskulatur versorgen, die zwar dauerleistungsfähig sind, jedoch nicht die Fähigkeit zur schnellen Kraftentwicklung besitzen, um auf die skoliotische Deformität im Sinne der Korrektur einzuwirken. Dies kann nur durch Vermehrung der sogenannten Zuckungsfasern erreicht werden.

Davon ausgehend ist es Aufgabe der vorliegenden Erfindung, ein Elektrostimulationsgerät anzugeben, das eine bessere Muskelkräftigung bei wesentlich kürzerer Behandlungszeit bewirkt. Diese Aufgabe wird mit einem in den Ansprüchen beschriebenen Gerät gelöst.

Die Erfindung wird im folgenden anhand einer

Zeichnung eines Ausführungsbeispiels näher erläutert.

Figur 1 zeigt das Ersatzschema der Impulsdeformierung durch verschiedene Gewebswiderstände,

Figur 2 zeigt einen Einzelimpuls des erfindungsgemäßen Gerätes,

Figur 3 zeigt eine Impulsserie des erfindungsgemäßen Gerätes und

Figur 4 zeigt das Blockschema der elektrischen Schaltung für das erfindungsgemäße Gerät.

Eingehende Untersuchungen haben gezeigt, daß von außen auf die Haut angelegte Impulse durch die verschiedenen Gewebsteile deformiert und insbesondere auch reduziert werden. Es ist möglich, die verschiedenen Gewebsteile in einer Ersatzschaltung als hintereinandergeschaltete ohmsche Widerstände und parallel geschaltete kapazitive Widerstände (Kondensatoren mit endlicher Isolation, d. h. mit Verlusten) darzustellen, die eine Reduzierung der ursprünglichen Amplitudenhöhe und eine Verminderung der Steilheit der Anstiegsflanken der Impulse bewirken. Dies wirkt sich insbesondere in tieferen Gewebsarealen aus.

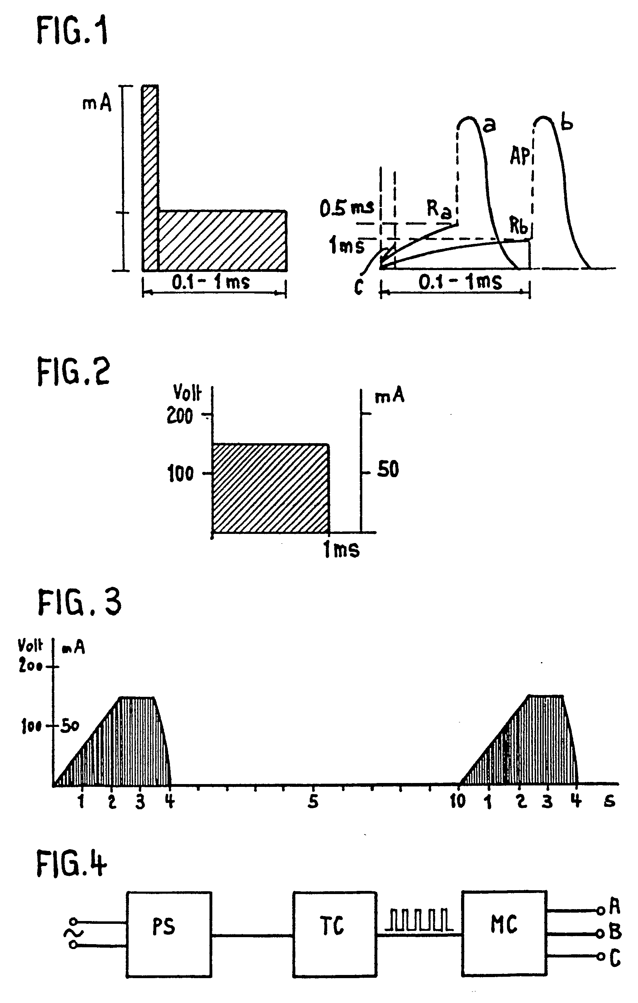

Der Rechteckimpuls mit senkrechter Flanke (siehe Fig. 1 und 2) ist die ideale Impulsform, um an erregbaren Membranen eine fortgeleitete Erregung auszulösen. Je rascher die Depolarisation erfolgt, um so schneller wird eine bestimmte Reizschwelle überwunden. Jede Verzögerung der Depolarisation hat eine Verminderung der Errregbarkeit, d. h. eine Erhöhung der Reizschwelle, zur Folge. Dies gilt auch für die Dauer der Einwirkung der Depolarisation. Je länger ein depolarisierender Strom fließt, um so niedriger ist die Reizschwelle, je kürzer ein depolarisierender Strom einwirkt, um so höher ist die Reizschwelle. Dies kann anhand der schematisch dargestellten Impulsverformungen gemäß Figur 1 dargestellt werden. Der Rechteckimpuls ist die Impulsform, die auch in tiefer gelegenen Gewebsarealen die größte Anstiegssteilheit aufweist. Der ausreichend lange Einzelimpuls mit einer Dauer von ungefähr einer ms beinhaltet die niedrigste Reizschwelle Rb zur Auslösung einer fortgeleiteten Erregung, siehe Kurve b. Ein kürzerer Impuls, beispielsweise unterhalb 0,5 ms, hat eine höhere Reizschwelle Ra zur Folge, so daß ein stärkerer Einzelimpuls von außen appliziert werden muß, um eine fortgeleitete Erregung zu erzeugen, siehe Kurve a.

Bereits vordeformierte Einzelimpulse, wie sie bei den vorbekannten Geräten ausnahmslos vorkommen, werden durch die ohmschen bzw. kapazitiven Gewebswiderstände zusätzlich deformiert, so daß in der Gewebstiefe, in der auch vorrangig eine Erregung erforderlich ist – tiefe Muskelschichten der paravertebralen Muskulatur bzw. Interkostalnerven – keine ausreichende Anstiegssteilheit bzw. Stromamplitude und Plateaudauer zur Verfügung steht, um eine fortgeleitete Erregung auszulösen, siehe Kurve c. In der Literatur, bzw. den weiter oben angegebenen amerikanischen Patentschriften, wird wohl erwähnt, daß Rechteckimpulse wünschenswert wären aber eine gewisse Deformierung derselben in Kauf genommen werden kann. Die das Ersatzschema sowie die Impulsdeformierungskurven bestätigenden Untersuchungen haben jedoch gezeigt, daß auf eine strenge und möglichst ideale Rechteckform der Impulse geachtet werden muß. Um eine Erregung auszulösen, ist ferner eine Mindeststromamplitude erforderlich (Rheobase), denn unterhalb dieser Amplitude ist ein depolarisierender Impuls nicht wirksam. Bei der geringen Stromstärke, die appliziert werden darf, sind relativ lange Impulse erforderlich, so bei Muskel- bzw. motorischen Nervenmembranen mit einer Dauer von etwa einer ms. Um in tieferen Gewebsarealen eine ausreichende Sicherheitsreserve zu haben, ist ein Strom von mindestens 50 mA und eine Impulsdauer von mindestens 0,5 ms erforderlich. Die erforderliche Amplitudenhöhe hängt von der Größe der Gewebswiderstände ab, die während der Untersuchung ermittelt wurden.

Ausgehend von diesen Erkenntnissen und den eingangs erwähnten Aufgaben, einen optimalen Erfolg bei kürzester Zeitdauer zu erzielen, d. h. vor allem die Zahl der Tonusfasern zurückzudrängen und das gestörte Gleichgewicht zu Gunsten der Zuckungsfasern neu einzustellen und ein sogenanntes Maximaltraining durchzuführen, da beim Training mit maximaler Muskelanspannung bei nur kurzer Trainingszeit ein wesentlich größerer Trainingseffekt erzielt wird als beim langfristigen Dauertraining mit niedriger Muskelanspannung, ist ein Gerät vorzusehen, das folgende Stromparameter liefern kann:

1. Rechteckimpulse mit möglichst steil ansteigenden Flanken, mit einer Impulsbreite von 0,5–5 ms, vorzugsweise 1 ms,

2. eine Amplitude der Einzelimpulse von 100–200 V, vorzugsweise 150 V, was bei entsprechendem Gewebswiderstand und Regelung einer Stromstärke von mindestens 50 mA entspricht, um eine maximale Eindringtiefe zu erreichen,

3. eine Impulsfrequenz von 75–125 Hz, vorzugsweise 100 Hz, zur Normalisierung der Fasertypenzusammensetzung,

4. eine Impulsserie von mindestens 2 bis vorzugsweise und maximal 4 s mit einem Anstieg der Impulsamplituden über einen Zeitraum von etwa 2 s und einer Plateauphase von 1–2 s (siehe Figur 3),

5. drei miteinander synchronisierte Kanäle, deren Amplitude jedoch individuell eingestellt werden kann und

6. Mittel, um die einmal eingestellte Stromstärke konstant zu halten um zu verhindern, daß sich bei ändernden Widerständen die Stromstärke verändert.

In Figur 4 erkennt man ein vereinfachtes Blockschema eines solchen Elektrostimulationsgerätes. Das am Netz angeschlossene Netzteil PS ist vorgesehen, eine konstante Spannung von plus und minus 15 V sowie eine regelbare Spannung von 0–200 V zu liefern und enthält einen Stromkonstanthalter, in welchem eine einstellbare Stromstärke von 50–100 mA konstant gehalten werden kann. Das Netzteil ist mit einer Zeitschaltung TC verbunden, in welchem Rechteckimpulse von beispielsweise 1 ms und einer Frequenz von 100 Hz erzeugt werden, wobei die Amplitude der Rechteckimpulse variabel ist. Selbstverständlich können auch andere Impulse mit anderen Frequenzen im bereits genannten Bereich erzeugt werden. Die Impulse gelangen in eine Modulator- und Stromverstärkungsschaltung MC, in welcher sie zu Impulsserienfolgen geformt werden, wobei die Amplitude beispielsweise innerhalb 2 s von 0 auf 150 V steigt, anschließend eine Plateauphase von 2 s aufrechterhalten wird, gefolgt von einem Abstieg und einer anschließenden Pause von 10 ms, wie dies in der Figur 3 dargestellt ist. Dabei wird die Stromstärke, wie bereits erwähnt, konstant auf beispielsweise 75 mA gehalten. Die Impulsfolgen können synchron auf allen drei Kanälen A, B und C abgegeben werden. Dabei gelingt es, aufgrund der besonderen Impulsform und -dauer, von einer Elektrodenposition aus alle für die Korrektur der Skoliose bedeutungsvollen Muskelgruppen einer Auskrümmung gleichzeitig zu stimulieren. Wird nun die erste Elektrode (Kanal) auf eine Auskrümmung, die zweite Elektrode auf die in den meisten Fällen vorhandene Gegenkrümmung, und die dritte Elektrode auf die schräge Bauchmuskulatur angesetzt, wird ein sehr schneller und gründlicher Heilungserfolg erzielt.

Bei der Mehrzahl der Geräte ist es vorteilhaft, die Serien- und Pausendauer festzulegen und auch die Dauer der Einzelimpulse sowie deren Wiederholungsfrequenz konstant zu halten. Es kann jedoch für gewisse Anwendungen von Vorteil sein, sämtliche Parameter innerhalb gewisser Grenzen zu verändern und insbesondere auch gewisse Parameter der verschiedenen Kanäle unabhängig voneinander zu variieren. Insbesondere ist bei einem solchen Gerät darauf zu achten, daß möglichst ideale Rechteckimpulse, d. h. mit möglichst steiler Anstiegsflanke, erzeugt werden, da die Impulse, wie in Figur 1 gezeigt, in der Haut stark deformiert werden. Behandlungen mit einem solchen Gerät, d. h. mit einem sehr guten Rechteckimpuls von 1 ms Dauer und einer Frequenz von 100 Hz sowie den Impulsserien gemäß Figur 3, d. h. mit einer Plateauamplitude von 150 V und einer konstanten Stromstärke von 75 mA, ermöglichen Behandlungszeiten von 20–30 Min. pro Tag, im Gegensatz zu Behandlungszeiten von bis zu 16 Stunden mit vorbekannten Geräten. Dadurch kann die Behandlung am Tage, beispielsweise nach der Schule oder abends vor dem Schlafengehen, erfolgen und die Nachtruhe wird nicht gestört. Außerdem kann eine Hautreizung auf Grund der kurzen Applikationszeit vermieden werden.

**Patentansprüche**

1. Elektrostimulationsgerät, insbesondere zur Skoliosebehandlung, mit mindestens einem Kanal für ein Elektrodenpaar und einer elektrischen Schaltung zur Einstellung der Dauer, Amplitude und Frequenz der Einzelimpulse sowie der Pausen zwischen den Einzelimpulsen und des zeitlichen Verlaufs von Impulsserien, wobei die Amplitude der einzelnen Rechteckimpulse in einer Anfangsphase zunimmt, dann einen Plateauwert annimmt und anschließend wieder abnimmt, wobei die elektrische Schaltung (TC, MC) Einzelimpulse mit einer Dauer von 0,5–5 ms, einer Amplitude von 100–200 V, einer konstanten Stromstärke von 50–100 mA und einer Frequenz von 75–125 Hz sowie Impulsserien mit einer Anstiegsphase von 1–2 s und einer Plateauphase von 1–2 s liefert, wobei die Pausen zwischen zwei Impulsserien 8–12 s betragen.

2. Gerät nach Anspruch 1, dadurch gekennzeichnet, daß es drei miteinander synchronisierte Kanäle (A, B, C) besitzt, wobei je ein Kanal für die Krümmungsseite und Gegenkrümmungsseite und der dritte Kanal für die Bauchmuskulatur vorgesehen ist.

3. Gerät nach Anspruch 1 oder 2, dadurch gekennzeichnet, daß die elektrische Schaltung (TC, MC) Einzelimpulse mit einer Dauer von ca. 1 ms, einer Amplitude von ca. 150 V, einer konstanten Stromstärke von ca. 75 mA und einer Frequenz von ca. 100 Hz sowie Impulsserien mit einer Anstiegsphase von 2 s und einer anschließenden Plateauphase von 2 s und Pausen zwischen zwei Impulsserien von 10 s liefert.

**Claims**

1. Apparatus for electrical stimulation, more particularly for the treatment of scoliosis, comprising at least one channel for a pair of electrodes and an electrical circuit for adjusting the duration, amplitude and frequency of individual pulses as well as the time off between the individual pulses and the evolution in time of series of pulses, wherein the amplitude of the individual square pulses increases in an initial phase, then reaches a plateau level, and finally decreases again, wherein the electrical circuit (TC, MC) delivers individual pulses having a duration of 0.5–5 ms, an amplitude of 100–200 V, a constant current of 50–100 mA intensity and a frequency of 75–125 Hz as well as series of pulses with a rising periods of 1–2 s and a plateau period of 1–2 s, whereby the time offs between two series of pulses are of 8–12 s.

2. Apparatus according to claim 1, characterised in that it has three channels (A, B, C), one channel being provided for the side of the curvature and each for the side of the counter curvature respectively, the third channel being provided for the abdominal musculature.

3. Apparatus according to claim 1 or 2, characterised in that said electrical circuit (TC, MC) delivers individual pulses with a duration of about 1 ms, an amplitude of about 150 V, a constant intensity of the current of about 75 mA and a frequency of about 100 Hz as well as series of pulses with a rising period of 2 s and a following plateau period of 2 s duration and time offs of 10 s between two series of pulses.

## Revendications

1. Appareil d'électrostimulation, en particulier pour le traitement de la scoliose, avec au moins un canal pour une paire d'électrodes et un circuit électrique pour l'ajustement de la durée, de l'amplitude et de la fréquence des impulsions individuelles ainsi que des intervalles entre les impulsions individuelles et du dévoulement dans le temps de séries d'impulsions, l'amplitude des impulsions rectangulaires individuelles croissant au cours d'une phase initiale, atteignant ensuite une valeur-plateau et finalement décroissant de nouveau, le circuit électrique (TC, MC) fournissant des impulsions individuelles d'une durée de 0,5 à 5 ms, d'une amplitude de 100 à 200 V, de courant constant de 50 à 100 mA et d'une fréquence de 75 à 125 Hz ainsi que des séries d'impulsions dont la période de croissance est de 1 à 2 s et la phase-plateau de 1 à 2 s, les intervalles entre deux séries d'impulsions étant de 8 à 10 s.

2. Appareil suivant la revendication 1, caractérisé en ce qu'il comporte trois canaux synchronisés (A, B, C), un canal chacun étant prévu pour le côté de la déviation et le côté opposé à la déviation respectivement, et le troisième pour la musculature abdominale.

3. Appareil suivant la revendication 1 ou 2, caractérisé en ce que le circuit électrique (TC, MC) fournit des impulsions individuelles d'une durée d'environ 1 ms, d'une amplitude d'environ 150 V, de courant constant d'environ 75 mA et d'une fréquence d'environ 100 Hz, ainsi que des séries d'impulsions dont la période de montée est de 2 s et la phase-plateau qui suit de 2 s et des intervalles de 10 s entre deux séries d'impulsions.

FIG.1

FIG.2

FIG.3

FIG.4